(19) **Europäisches Patentamt / European Patent Office / Office européen des brevets**

(11) **EP 4 517 405 B1**

(12) **EUROPEAN PATENT SPECIFICATION**

(45) Date of publication and mention
of the grant of the patent:
**22.04.2026 Bulletin 2026/17**

(21) Application number: **23815589.9**

(22) Date of filing: **11.04.2023**

(51) International Patent Classification (IPC):
**G03H 1/04** (2006.01)    **G03H 1/08** (2006.01)
**G01N 21/45** (2006.01)    **G01N 33/08** (2006.01)
**G02B 21/14** (2006.01)    **G01N 15/01** (2024.01)
**G01N 15/1434** (2024.01)    **G03H 1/00** (2006.01)
**C12M 1/34** (2006.01)    **G01N 15/10** (2024.01)
**G01N 15/0227** (2024.01)    **G02B 21/32** (2006.01)
**G06T 7/194** (2017.01)

(52) Cooperative Patent Classification (CPC):
**G01N 21/453; G01N 15/01; G01N 15/1434;**
**G01N 33/08; G02B 21/14; G03H 1/0486;**
**G03H 1/0866;** C12M 41/36; G01N 15/0227;
G01N 2015/1006; G01N 2015/1454; G02B 21/32;
G03H 1/0443; G03H 2001/0038; G03H 2001/005;
(Cont.)

(86) International application number:
**PCT/JP2023/014780**

(87) International publication number:
**WO 2023/233827 (07.12.2023 Gazette 2023/49)**

(54) **PHASE IMAGE ACQUISITION METHOD AND QUANTITATIVE DATA ACQUISITION METHOD**

PHASENBILDERFASSUNGSVERFAHREN UND QUANTITATIVES
DATENERFASSUNGSVERFAHREN

PROCÉDÉ D'ACQUISITION D'IMAGE DE PHASE ET PROCÉDÉ D'ACQUISITION DE DONNÉES
QUANTITATIVES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **03.06.2022 JP 2022091056**

(43) Date of publication of application:
**05.03.2025 Bulletin 2025/10**

(73) Proprietor: FUJIFILM Corporation
**Tokyo 106-8620 (JP)**

(72) Inventors:
 • **YASUDA, Hideki**
  **Ashigarakami-gun, Kanagawa 258-8577 (JP)**
 • **ONOZAWA, Sho**
  **Ashigarakami-gun, Kanagawa 258-8577 (JP)**
 • **OSAKI, Ryusuke**
  **Ashigarakami-gun, Kanagawa 258-8577 (JP)**

 • **NAKAMURA, Sohichiro**
  **Ashigarakami-gun, Kanagawa 258-8577 (JP)**

(74) Representative: **Hoffmann Eitle**
**Patent- und Rechtsanwälte PartmbB
Arabellastraße 30
81925 München (DE)**

(56) References cited:
**EP-A1- 2 985 719      WO-A1-2015/178413
WO-A1-2018/163683    WO-A1-2019/150695
WO-A1-2019/150695    WO-A1-2019/176427
WO-A1-2019/202979**

EP 4 517 405 B1

- **LAI XIAOMIN ET AL: "Digital holographic phase imaging with aberrations totally compensated", vol. 10, no. 1, 1 January 2019 (2019-01-01), United States, pages 283, XP093206460, ISSN: 2156-7085, Retrieved from the Internet <URL:https://opg.optica.org/directpdfaccess/64c9d01a-c4aa-4d69-aeeaacd89cd1c05f_403263/boe-10-1-283.pdf?da=1&id=403263&seq=0&mobile=no> [retrieved on 20250703], DOI: 10.1364/BOE.10.000283**
- **SIRICO DANIELE GAETANO ET AL: "Compensation of aberrations in holographic microscopes: main strategies and applications", APPLIED PHYSICS B, SPRINGER BERLIN HEIDELBERG, BERLIN/HEIDELBERG, vol. 128, no. 4, 24 March 2022 (2022-03-24), XP037746565, ISSN: 0946-2171, [retrieved on 20220324], DOI: 10.1007/S00340-022-07798-8**

(52) Cooperative Patent Classification (CPC): (Cont.)
G03H 2210/12

**Description**

**BACKGROUND OF THE INVENTION**

1. Field of the Invention

The disclosed technology relates to a phase image acquisition method and a quantitative data acquisition method.

2. Description of the Related Art

The following technology is known as a technology for monitoring cells using a holography technology. For example, JP2018-516591A describes a cell culture incubator including an incubator cabinet including an internal chamber for incubating cells in a cell culture container and a holographic imager configured to image the cells in the internal chamber. The research article LAI XIAOMIN ET AL: "Digital holographic phase imaging with aberrations totally compensated" (BIOMEDICAL OPTICS EXPRESS, vol. 10, no. 1, 1 January 2019 (2019-01-01), page 283) describes a phase image acquisition method, wherein aberrations are fully compensated by applying a 1D fitting method.

**SUMMARY OF THE INVENTION**

A phase image generated based on a hologram image (interference image) formed by interference between object light transmitted through a cell and reference light coherent to the object light is an image showing a phase distribution of the object light transmitted through the cell, and a state of the cell is reflected. Accordingly, it is possible to perform quality evaluation of the cell based on the phase image. For example, a total phase amount obtained by integrating a phase amount for each pixel of the phase image can be used as quantitative data for evaluating the cell.

The phase image of the cell can be acquired in a state where the cell is accommodated in a concave portion (well) provided in a dish (container) with a bottom surface of the concave portion being inclined. In the phase image of the cell acquired in this state, a phase component derived from the inclination of the bottom surface of the concave portion is superimposed. The phase component derived from the bottom surface of the concave portion is, so to speak, noise, and causes a decrease in the accuracy of the quantitative data obtained from the phase image.

The disclosed technology has been made in view of the above points, and an object of the disclosed technology is to appropriately remove unnecessary phase components from the phase image of the object.

A phase image acquisition method according to the disclosed technology includes: acquiring a hologram image of an object in a state where the object is accommodated in a concave portion provided in a container with a bottom surface of the concave portion being curved; generating a phase image from the hologram image; and performing processing of subtracting a phase component derived from a polynomial indicating a shape of the bottom surface of the concave portion from the phase image to acquire a processed phase image.

It is preferable that a degree of the polynomial is 2 or more and 7 or less. It is preferable that a change amount in a height of the bottom surface of the concave portion in the phase image is 10 $\mu$m or more.

The object may be, for example, a cell or a cell aggregate. The cell or the cell aggregate may be a fertilized egg of an animal.

The phase image acquisition method according to an embodiment of the disclosed technology may further include: acquiring a phase image of the cell or the cell aggregate cultured in a state of being accommodated in the concave portion.

A quantitative data acquisition method according to the disclosed technology is a method for acquiring quantitative data using the above-described phase image acquisition method, the quantitative data acquisition method includes: deriving quantitative data indicating a state of the cell or the cell aggregate based on the processed phase image.

According to the disclosed technology, it is possible to appropriately remove unnecessary phase components from the phase image of the object.

**BRIEF DESCRIPTION OF THE DRAWINGS**

Fig. 1 is a diagram showing an example of a culture state of a fertilized egg according to an embodiment of the disclosed technology.
Fig. 2 is a diagram showing an example of a configuration of a holography apparatus according to the embodiment of the disclosed technology.

Fig. 3 is a diagram showing an example of a phase image before unwrapping of the fertilized egg according to the embodiment of the disclosed technology.

Fig. 4 is a diagram showing a concept of the phase image according to the embodiment of the disclosed technology.

Fig. 5 is a diagram showing an example of function fitting performed on a shape of a bottom surface of a concave portion according to the embodiment of the disclosed technology.

Fig. 6A is a diagram showing an example of a processed phase image according to the embodiment of the disclosed technology.

Fig. 6B is a diagram showing an example of a processed phase image according to the embodiment of the disclosed technology.

Fig. 6C is a diagram showing an example of a processed phase image according to the embodiment of the disclosed technology.

Fig. 7 is a diagram showing an example of a configuration of a container according to a comparative example.

Fig. 8 is a diagram showing a change amount in a height of the bottom surface of the concave portion according to the embodiment of the disclosed technology.

## DESCRIPTION OF THE PREFERRED EMBODIMENTS

Hereinafter, an example of embodiments of the disclosed technology will be described with reference to the drawings. It should be noted that the same or equivalent components and portions in the drawings are assigned by the same reference numerals, and the overlapping description will be omitted.

A phase image acquisition method according to an embodiment of the disclosed technology includes: a first step of acquiring a hologram image of an object in a state where the object is accommodated in a concave portion provided in a container with a bottom surface of the concave portion being curved; a second step of generating a phase image from the hologram image; and a third step of performing processing (background removal processing) of subtracting a phase component derived from a polynomial according to a shape of the bottom surface of the concave portion from the phase image to acquire a processed phase image.

Each of the first to third steps will be described in detail below. In the present embodiment, a case where the object to be acquired as the phase image is a fertilized egg of an animal will be described as an example. The fertilized egg may be developed to a state of an embryo. The phase image acquisition method according to the disclosed technology can be used, for example, for the purpose of quality management, quality evaluation, quality inspection, or quality prediction of the fertilized eggs.

<First Step: Acquisition of Hologram Image>

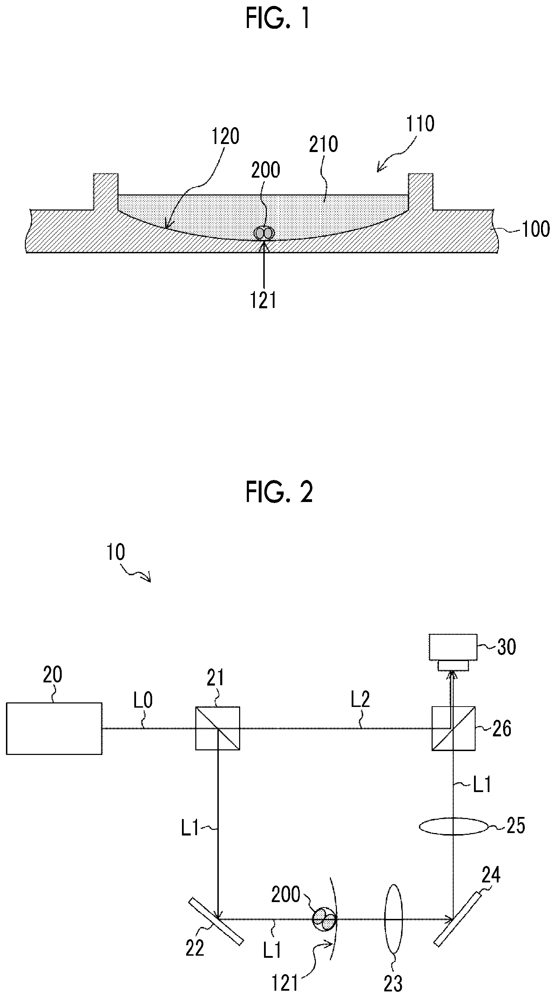

Fig. 1 is a diagram showing an example of a culture state of a fertilized egg 200. The fertilized egg 200 is cultured in a state of being accommodated in a concave portion 110 provided in a container 100. The container 100 may include a plurality of the concave portions 110, and the fertilized eggs 200 may be accommodated in each of the plurality of concave portions 110. The container 100 may be, for example, a commercially available dish or petri dish. The concave portion 110 may be a well provided in a dish or a petri dish. As the container 100, for example, 38Special GPS (registered trademark) manufactured by CooperSurgical, Inc. can be suitably used.

Culture medium 210 is accommodated in the concave portion 110 together with the fertilized egg 200, and the fertilized egg 200 is cultured in a state of being immersed in the culture medium 210 in the concave portion 110. The bottom surface 120 of the concave portion 110 is curved of which the height at a center portion 121 is the lowest and is inclined in a direction that gently rises from the center portion 121 toward the outside. The shape of the bottom surface 120 of the concave portion 110 may be a spherical shape.

In a case of acquiring a hologram image of the fertilized egg 200, it is necessary to capture the fertilized egg 200 with a microscope. It is preferable to suppress the movement amount of the fertilized egg 200 in order to maintain a state where the fertilized egg 200 is within the visual field of the microscope. In a case where the fertilized egg 200 is disposed on a flat surface, the fertilized egg 200 swings due to convection or the like caused by heating of the culture medium, and it is difficult to maintain the state where the fertilized egg 200 is within the visual field of the microscope. Since the bottom surface 120 of the concave portion 110 has an inclination such that the height at the center portion 121 is the lowest, the fertilized egg 200 can be disposed in the vicinity of the center portion 121, and the movement amount of the fertilized egg 200 can be suppressed.

In the first step, the hologram image of the fertilized egg 200 is acquired. The hologram image of the fertilized egg 200 is acquired in a state where the fertilized egg 200 is accommodated in the concave portion 110. Fig. 2 is a diagram showing an example of a configuration of a holography apparatus 10 used for acquiring the hologram image.

The holography apparatus 10 includes a splitter 21, reflection mirrors 22 and 24, an objective lens 23, an imaging lens 25, a combiner 26, and an imaging apparatus 30. The fertilized egg 200 as the imaging target is disposed between the reflection mirror 22 and the objective lens 23 in a state of being accommodated in the concave portion 110 of the container 100 together with the culture medium.

For example, a HeNe laser having a wavelength of 632.8 nm can be used as a laser light source 20. Laser light L0 which linearly polarized light emitted from the laser light source 20 is divided into two laser light rays by the splitter 21. One of the two laser light rays is object light L1 and the other is reference light L2. A beam splitter can be used as the splitter 21. The object light L1 is incident on the reflection mirror 22. The fertilized eggs 200 are irradiated with the object light L1 of which a traveling direction is bent by the reflection mirror 22. The object light L1 is irradiated in a direction that penetrates the bottom surface 120 of the concave portion 110.

An image formed by the object light L1 transmitted through the fertilized egg 200 is magnified by the objective lens 23. The object light L1 transmitted through the objective lens 23 is bent in a traveling direction by the reflection mirror 24 and is incident on the combiner 26 through the imaging lens 25. On the other hand, the reference light L2 is also incident on the combiner 26. The object light L1 and the reference light L2 are combined by the combiner 26 and are imaged on an imaging surface of the imaging apparatus 30. A beam splitter can be used as the combiner 26.

The hologram image, which is the interference image generated by the interference between the object light L1 and the reference light L2, is imaged by the imaging apparatus 30. The imaging apparatus 30 comprises an imaging element such as a complementary metal-oxide-semiconductor (CMOS) image sensor, and generates image data of the hologram image.

<Second Step: Acquisition of Phase Image>

An example of a method for acquiring the phase image from the hologram image will be described below. First, the hologram image of the fertilized egg 200 acquired by the imaging apparatus 30 is trimmed to have a size of, for example, $2048 \times 2048$, and is then subjected to a two-dimensional Fourier transform. A Fourier-transformed image obtained by this processing may include an image based on direct light, object light, and conjugated light.

Subsequently, a position of the object light is specified by specifying a deviation amount of the object light with respect to the direct light in the Fourier-transformed image, and, for example, a complex amplitude component of only the object light is extracted by frequency filtering processing using a mask of a circular opening having a radius of 250 pixels.

Subsequently, for example, an angular spectrum method is applied to restore the image showing the phase of the fertilized egg 200 at any spatial position. Specifically, an angular spectrum $U(f_x, f_y; 0)$ of the Fourier-transformed image of a wave front $u(x, y; 0)$ captured by the imaging surface of the imaging apparatus 30 is obtained. Subsequently, as represented in Equation (1) below, the angular spectrum $U(f_x, f_y; 0)$ is multiplied by a transfer function $H(f_x, f_y; z)$, and thus, a wave front at any position z in an optical axis direction (z direction) is reproduced. Here, the transfer function $H(f_x, f_y; z)$ is a frequency response function (Fourier transform of an impulse response function (green function)).

$$U\left(f_x, f_y; z\right) = U\left(f_x, f_y; 0\right)H\left(f_x, f_y; z\right), \quad H = e^{z\frac{2\pi}{\lambda}\sqrt{1-(\lambda f_x)^2-(\lambda f_y)^2}} \quad \cdots (1)$$

Subsequently, as represented in Equation (2) below, an inverse Fourier transform is performed on a wave front $U(f_x, f_y; z)$ at the position z in the optical axis direction (z direction), and thus, a solution $u(x, y; z)$ at the position z is derived.

$$\begin{aligned} u\left(x, y; z\right) &= F^{-1}\left[U\left(f_x, f_y; z\right)\right] \\ &= F^{-1}\left[U\left(f_x, f_y; 0\right)H\left(f_x, f_y; z\right)\right] \quad \cdots (2) \\ &= F^{-1}\left[F\left[u(x, y; 0)\right]H\left(f_x, f_y; z\right)\right] \end{aligned}$$

Subsequently, the phase image is generated by deriving a phase φ for $u(x, y; z)$ as represented in Equation (3) below.

$$\phi = \arctan\left(\frac{\mathrm{Im}(u)}{\mathrm{Re}(u)}\right) \quad \cdots (3)$$

A phase in the phase image before unwrapping obtained by the above processing is convolved to a value of 0 to

$2\pi$. Therefore, for example, a phase connection (unwrapping) method such as unweighted least squares or Flynn's algorithm is applied to connect the portions of $2\pi$ or more. It should be noted that, many unwrapping methods have been proposed, and an appropriate method that does not cause phase mismatch may be appropriately selected.

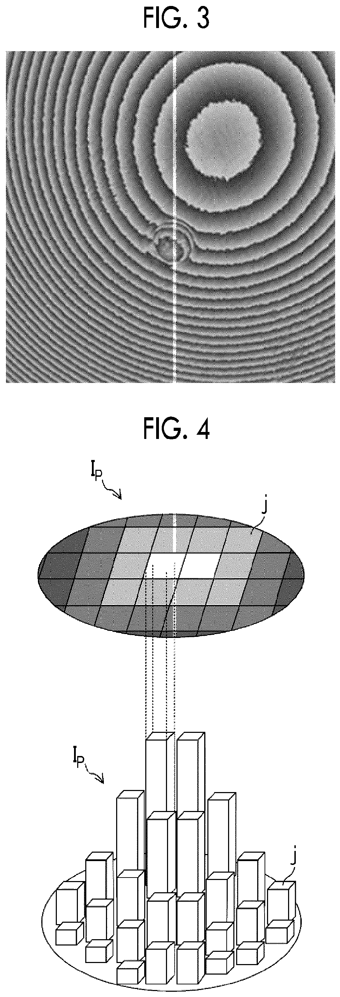

Fig. 3 is a diagram showing an example of a phase image before unwrapping of the fertilized egg 200. A phase component derived from the inclination of the bottom surface 120 of the concave portion 110 is superimposed on the phase image. The phase component derived from the bottom surface 120 of the concave portion 110 is, so to speak, noise, and causes a decrease in the accuracy of the quantitative data obtained from the phase image. The phase component derived from the bottom surface 120 of the concave portion 110 is removed by the background removal processing described below.

<Third Step: Removal of Background>

Fig. 4 is a diagram showing a concept of a phase image $I_P$. In a lower part of Fig. 4, a phase amount at each pixel j of the phase image $I_P$ is three-dimensionally displayed. In an upper part of Fig. 4, the phase amount at each pixel j of the phase image $I_P$ is shown on a plane in gray scale.

Here, in a case where the phase amount at each pixel j of the phase image $I_P$ is denoted by $P_S$ and the phase of the background (region where the fertilized egg 200 is not present) present in the same focal plane is denoted by $P_B$, the background removal processing can be represented by Equation (4) below. The term "phase" in the present specification is a phase of an electric field amplitude in a case where light is regarded as an electromagnetic wave, and is used in a more general sense.

$$P = P_S - P_B \quad \cdot \cdot \cdot (4)$$

The phase $P_B$ of the background is a phase component derived from the bottom surface 120 of the concave portion 110. Therefore, the phase $P_B$ of the background can be calculated by specifying the shape of the bottom surface 120 of the concave portion 110. Since the bottom surface 120 of the concave portion 110 is curved, it is possible to perform function fitting with a degree of a polynomial of 2 or more on the shape of the bottom surface 120 of the concave portion 110. The function fitting can be performed, for example, by acquiring a profile of the shape of the bottom surface 120 of the concave portion 110 by actual measurement and searching for a polynomial approximating the acquired profile.

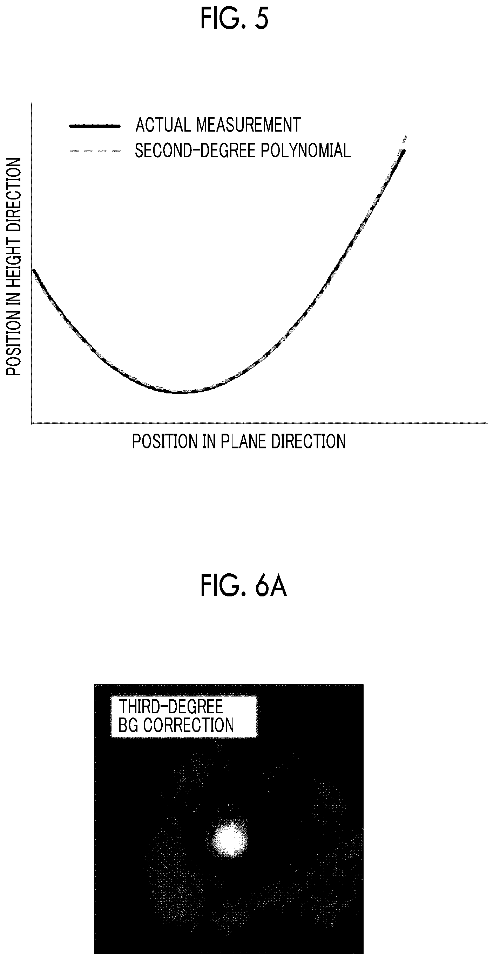

Fig. 5 is a diagram showing an example of function fitting performed on the shape of the bottom surface 120 of the concave portion 110. In Fig. 5, a horizontal axis indicates a position of the concave portion 110 in the plane direction, and a vertical axis indicates a position of the concave portion 110 in the height direction. In Fig. 5, a dotted line is a curve defined by a second-degree polynomial indicating the shape of the bottom surface 120 of the concave portion 110, and a solid line is a profile obtained by actually measuring the shape of the bottom surface 120 of the concave portion 110. It can be seen that the solid line and the dotted line substantially coincide with each other. As described above, the shape of the bottom surface 120 of the concave portion 110 can be described by a degree of a polynomial of 2 or more.

The phase $P_B$ of the background, that is, the phase component derived from the bottom surface 120 of the concave portion 110 can be derived from a polynomial indicating the shape of the bottom surface 120 of the concave portion 110. The phase $P_B$ of the background is represented by Equation (5) below. In Equation (5), x is a distance from the center portion 121 of the concave portion 110. m is a degree of the polynomial. $a_k$ is a coefficient of each term and can be obtained from the polynomial indicating the shape of the bottom surface 120 of the concave portion 110.

$$P_B = \sum_{k=0}^{m} a_k \cdot x^k \quad \cdot \cdot \cdot \quad (5)$$

In a case where the polynomial indicating the shape of the bottom surface 120 is denoted by C(x), the phase $P_B$ of the background can be converted by performing the conversion processing represented by Equation (6) below. In Equation (6), $n_s$ is a refractive index of the container 100, $n_m$ is a refractive index of the culture medium, and $\lambda$ is a wavelength of the object light in the hologram optical system.

$$P_B(x) = (n_s - n_m) \times \left(\frac{C(x)}{\lambda}\right) \times 2\pi \quad \cdots \quad (6)$$

In the background removal processing, as represented in Equation (4), the phase component derived from the polynomial indicating the shape of the bottom surface 120 of the concave portion 110 is subtracted from the phase amount of each pixel in the phase image obtained in the second step. As a result, a phase image from which the phase component derived from the bottom surface 120 of the concave portion 110 is removed is acquired. The phase $P_B$ of the background, that is, the phase image from which the phase component derived from the bottom surface 120 of the concave portion 110 is removed will be referred to as a "processed phase image" below.

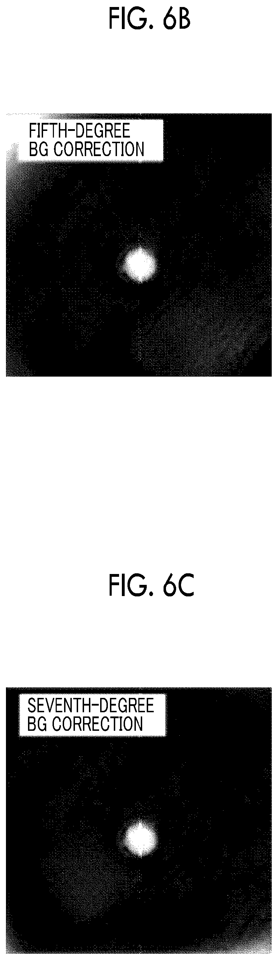

Figs. 6A, 6B, and 6C are diagrams showing examples of the processed phase images. Fig. 6A is a processed phase image in a case where the function fitting is performed with the degree of the polynomial indicating the shape of the bottom surface 120 of the concave portion 110 set to 3. Fig. 6B is a processed phase image in a case where the function fitting is performed with the degree of the polynomial indicating the shape of the bottom surface 120 of the concave portion 110 set to 5. Fig. 6C is a processed phase image in a case where the function fitting is performed with the degree of the polynomial indicating the shape of the bottom surface 120 of the concave portion 110 set to 7. As shown in Figs. 6A to 6C, by performing the background removal processing, a processed phase image from which the phase component derived from the bottom surface 120 of the concave portion 110 is removed and only the fertilized egg 200 is extracted can be acquired.

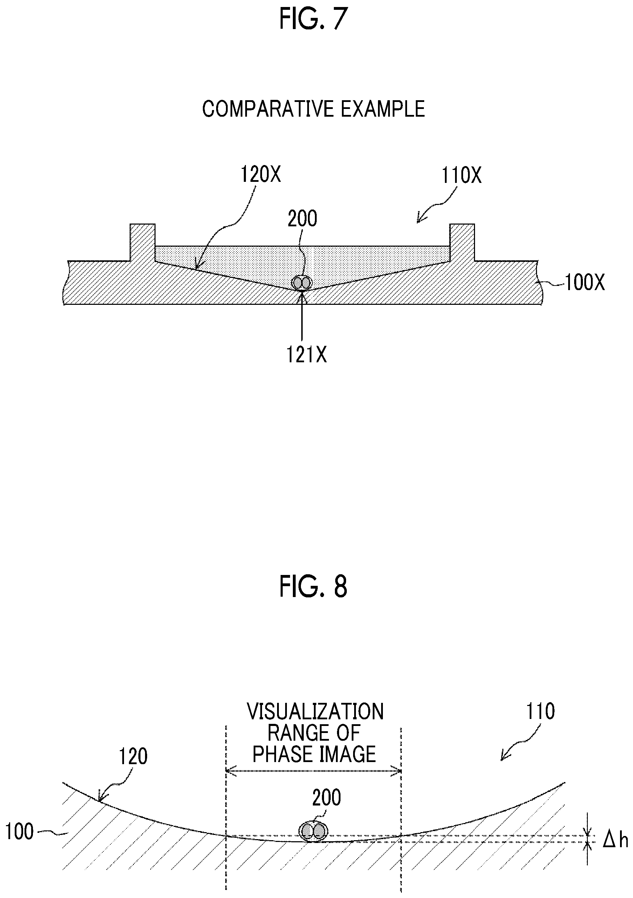

Fig. 7 is a diagram showing an example of a container 100X according to a comparative example. The container 100X is different from the container 100 (see Fig. 1) according to the embodiment of the disclosed technology in that the bottom surface 120X of a concave portion 110X is non-curved. That is, the inclination of the bottom surface 120X of the concave portion 110X according to the comparative example is linear.

According to the container 100X of the comparative example, since the change in height of a center portion 121X of the bottom surface 120X of the concave portion 110X is rapid, the error is large in a case where the function fitting is performed on the shape of the bottom surface 120X. Since the error in the function fitting is an error of the phase $P_B$ of the background derived based on the polynomial, in a case where the container 100X according to the comparative example is used, it is difficult to appropriately remove the phase component derived from the bottom surface 120X of the concave portion 110X.

On the other hand, since the bottom surface 120 of the concave portion 110 of the container 100 according to the embodiment of the disclosed technology is a curved surface of which the height at the center portion 121 is the lowest and is gently inclined toward the outside from the center portion 121, it is possible to perform high-accuracy function fitting on the shape of the bottom surface 120 of the concave portion 110 (see Fig. 5). That is, according to the phase image acquisition method according to the embodiment of the disclosed technology, it is possible to appropriately remove unnecessary phase components from the phase image of the object.

It is preferable that the degree of the polynomial indicating the shape of the bottom surface 120 of the concave portion 110 is 2 or more and 7 or less. By setting the degree of the polynomial to 2 or more, it is possible to describe the curved surface by the polynomial. By setting the degree of the polynomial to 7 or less, it is possible to perform high-accuracy function fitting on the shape of the bottom surface 120 of the concave portion 110, and it is possible to appropriately remove the phase component derived from the bottom surface 120 of the concave portion 110. In a case where the degree of the polynomial is 8 or more, an error tends to occur in the function fitting. It should be noted that, even in a case where the fitting is performed with the degree of 8 or more, in a case where the contribution of the coefficient ($a_k \times x^k$) is smaller than 10% of the sum ($\Sigma k = 2$ to $7$ $a_k \times x^k$) of the contributions of the coefficients of the degree of 2 to 7, it is assumed that the fitting is substantially performed with the degree of 7. In addition, it is preferable that the change amount $\Delta h$ (see Fig. 8) in the height of the bottom surface 120 of the concave portion 110 in the phase image (within the visualization range of the phase image) is 10 $\mu$m or more. In a case where the change amount $\Delta h$ is 10 $\mu$m or more, it is possible to perform the function fitting of the shape of the bottom surface 120 of the concave portion 110 with high accuracy, and it is possible to appropriately remove the phase component derived from the bottom surface 120 of the concave portion 110.

<Acquisition of Quantitative Data>

The method of deriving quantitative data according to the embodiment of the disclosed technology uses the above-described phase image acquisition method. That is, the quantitative data derivation method is a method of deriving the quantitative data indicating the state of the fertilized egg 200 based on the processed phase image acquired by performing the above-described phase image acquisition method. The quantitative data derivation method according to the present embodiment will be described in detail below.

The phase amount $P_j$ at each pixel j of the processed phase image can be represented by Equation (7) below.

Here, $n_j$ is a refractive index of the fertilized egg 200 in a part corresponding to each pixel j of the processed phase image, $d_j$ is a thickness of the fertilized egg 200 in the part corresponding to each pixel j of the processed phase image, and $\lambda$ is a wavelength of the object light in the hologram optical system.

$$P_j = 2\pi \frac{n_j \cdot d_j}{\lambda} \quad \cdot \cdot \cdot \quad (7)$$

The processed phase image of the fertilized egg 200 is an image showing a phase distribution of the object light L1 transmitted through the fertilized egg 200, and is also an image showing an optical path length distribution of the object light transmitted through the fertilized egg 200. Since the optical path length in the fertilized egg 200 corresponds to the product of the refractive index of the fertilized egg 200 and the thickness of the fertilized egg 200, the processed phase image of the fertilized egg 200 includes information on the refractive index and the thickness (shape) of the fertilized egg 200 as represented in Equation (7). Since the processed phase image of the fertilized egg 200 reflects the internal state of the fertilized egg 200, it is possible to perform quality evaluation of the fertilized egg 200 based on the processed phase image. Specifically, the total phase amount $P_A$ represented by Equation (8) below can be used as the quantitative data indicating the state of the fertilized egg 200. In Equation (8), s is an area of each pixel j of the processed phase image, and $v_j$ is a volume of the fertilized egg 200 in the part corresponding to each pixel j of the processed phase image. As represented in Equation (8), the total phase amount $P_A$ corresponds to the amount obtained by integrating the phase amount $P_j$ for each pixel of the processed phase image for all the pixels j. The pixel value of the processed phase image corresponds to the phase amount $P_j$.

$$P_A = \sum_{j=0}^{N} P_j \cdot s = \frac{2\pi}{\lambda} \sum_{j=0}^{N} n_j \cdot d_j \cdot s = \frac{2\pi}{\lambda} \sum_{j=0}^{N} n_j \cdot v_j \quad \cdot \cdot \cdot \quad (8)$$

For example, the quality of the fertilized egg 200 can be determined by performing the threshold value determination on the total phase amount $P_A$ derived based on the processed phase image. By deriving the quantitative data indicating the state of the fertilized egg 200 based on the processed phase image acquired by performing the phase image acquisition method according to the embodiment of the disclosed technology, the accuracy of the quantitative data can be enhanced, and the evaluation of the fertilized egg 200 can be appropriately performed.

The quantitative data derived based on the phase image is not limited to the total phase amount $P_A$. For example, statistical values such as a maximum value, a minimum value, a median value, an average value, a variance, and a standard deviation of the pixel values (phase amounts $P_j$) of the processed phase images can also be used as the quantitative data. In addition, a phase density, which is a value obtained by dividing the total phase amount $P_A$ by the volume of the fertilized egg 200, can also be used as the quantitative data.

In the above description, a case where the object from which the phase image is acquired is a fertilized egg has been described as an example, but the present embodiment is not limited to this aspect. The object may be a cell or a cell aggregate other than the fertilized egg. For example, the disclosed technology can be applied to a case of acquiring a phase image of an induced pluripotent stem cell (iPS cell) or a spheroid which is an aggregate thereof. In addition, the object from which the phase image is acquired may be an artificial object.

Examples

The mouse embryos are cultured for 5 days using the dish shown in Table 1 below. The shape of the bottom surface of the concave portion (well) provided in each dish is shown in Table 1. The bottom surface of the concave portion of the dish according to Examples 1 and 2 is curved as shown in Fig. 1. The dish according to Comparative Example 1 is a dish having a flat bottom surface without a concave portion. The bottom surface of the concave portion of the dish according to Comparative Example 2 is non-curved in which the inclination is linear as shown in Fig. 7. In a state where the mouse embryo is accommodated in each dish, a hologram image of the mouse embryo is acquired, and a phase image is acquired from the hologram image.

<Measurement of Change Amount Δh in Height of Bottom Surface>

For each dish, an image of a cross section is imaged, and a change amount Δh (see Fig. 8) in a height of a bottom surface of a concave portion (well) in the phase image is measured. The measured values of the change amount Δh are shown in Table 1.

<Background Removal Processing>

The phase images of the mouse embryos acquired in each of the examples and the comparative examples are subjected to background removal processing. In Examples 1 and 2 in which the bottom surface of the concave portion (well) is curved, the function fitting with a fifth-degree polynomial is performed on the shape of the bottom surface of the concave portion. In Comparative Example 2 in which the bottom surface of the concave portion is non-curved, the function fitting with a first-degree polynomial is performed on the shape of the bottom surface of the concave portion. A processed phase image is acquired by removing the phase component derived from these polynomials from the phase image. In Comparative Example 1 in which the bottom surface of the dish is flat, the background removal processing is not performed.

<Measurement of Movement Amount>

For each of the examples and the comparative examples, the movement amount of the mouse embryo during the culture period is measured. The position of the mouse embryo at the time of the start of the culture is defined as the initial position, and the distance from the initial position of the position of the mouse embryo in a case where the mouse embryo is moved to a position farthest from the initial position during the culture period is defined as the movement amount. The measured values of the movement amount are shown in Table 1. Since the dishes according to Example 1, Example 2, and Comparative Example 2 comprise the concave portion having the inclined bottom surface, the movement amount of the mouse embryo is suppressed. On the other hand, in a case where the dish according to Comparative Example 1 having a flat bottom surface is used, the movement amount of the mouse embryo is the largest.

<Measurement of Phase Measurement Error>

A phase measurement error is acquired for the processed phase images acquired for each of the examples and the comparative examples. The total phase amount $P_A$ represented by Equation (8) is repeatedly measured 100 times with respect to the same mouse embryo immediately after the start of the culture. A value obtained by dividing the width of the phase amount at which the variation of the total phase amount $P_A$ is $\pm 1\sigma$ by the average value of the total phase amount $P_A$ is defined as a phase measurement error. $\sigma$ is a standard deviation. The measured values of the phase measurement error are shown in Table 1.

In the dishes according to Examples 1 and 2 in which the bottom surface of the concave portion (well) is curved, it is possible to perform high-accuracy function fitting on the shape of the bottom surface of the concave portion, and the background removal processing can be appropriately performed. As a result, the phase measurement error could be set to 5% or less. On the other hand, in the dish according to Comparative Example 2 in which the shape of the bottom surface of the concave portion is non-curved, it is difficult to perform high-accuracy function fitting on the shape of the bottom surface of the concave portion, and it is difficult to appropriately perform the background removal processing. As a result, the phase measurement error is significantly larger compared to Examples 1 and 2.

As described above, by performing the background removal processing using the dishes according to Examples 1 and 2, it is possible to suppress the movement amount of the mouse embryo and to acquire quantitative data (total phase amount $P_A$) with a small error.

[Table 1]

| | Dish product name | Shape of bottom surface | Change amount Δh in height of bottom surface | Fitting function | Movement amount | Phase measurement error |
|---|---|---|---|---|---|---|
| Example 1 | 38Special GPS® manufactured by CooperSurgical, Inc. | Curved | 28 μm | fifth-degree polynomial | 0.1 mm | ±3.4% |

(continued)

|  | Dish product name | Shape of bottom surface | Change amount Δh in height of bottom surface | Fitting function | Movement amount | Phase measurement error |
|---|---|---|---|---|---|---|
| Example 2 | Embryo GPS® manufactured by CooperSurgical, Inc. | Curved | 5 μm | fifth-degree polynomial | 0.2 mm | ±4.5% |
| Comparative Example 1 | Falcon® Cell Culture Dish manufactured by Corning | Flat | 0 μm | - | 20 mm | ±3.2% |
| Comparative Example 2 | Manufactured by DNP Group | Non-curved | 20 μm | first-degree polynomial | 0.1 mm | ±10.5% |

## Claims

1. A phase image acquisition method comprising:

   acquiring a hologram image of an object in a state where the object is accommodated in a concave portion provided in a container with a bottom surface of the concave portion being curved;
   generating a phase image from the hologram image; and
   performing processing of subtracting a phase component derived from a polynomial indicating a shape of the bottom surface of the concave portion from the phase image to acquire a processed phase image.

2. The phase image acquisition method according to claim 1,
   wherein a degree of the polynomial is 2 or more and 7 or less.

3. The phase image acquisition method according to claim 2,
   wherein a change amount in a height of the bottom surface of the concave portion in the phase image is 10 μm or more.

4. The phase image acquisition method according to any one of claims 1 to 3,
   wherein the object is a cell or a cell aggregate.

5. The phase image acquisition method according to claim 4,
   wherein the cell or the cell aggregate is a fertilized egg of an animal.

6. The phase image acquisition method according to claim 4, further comprising:
   acquiring a phase image of the cell or the cell aggregate cultured in a state of being accommodated in the concave portion.

7. A quantitative data acquisition method using the phase image acquisition method according to claim 4, the quantitative data acquisition method comprising:
   deriving quantitative data indicating a state of the cell or the cell aggregate based on the processed phase image.

## Patentansprüche

1. Phasenbild-Erfassungsverfahren, umfassend:

   Erfassen eines Hologrammbildes eines Objekts in einem Zustand, in dem das Objekt in einem in einem Behälter vorgesehenen konkaven Abschnitt aufgenommen ist, wobei eine Bodenfläche des konkaven Abschnitts gekrümmt ist;
   Erzeugen eines Phasenbildes aus dem Hologrammbild; und
   Durchführen von Verarbeitung des Subtrahierens einer Phasenkomponente, die aus einem Polynom, das eine Form der Bodenfläche des konkaven Abschnitts angibt, abgeleitet ist, von dem Phasenbild, um ein verarbeitetes Phasenbild zu erfassen.

**2.** Phasenbild-Erfassungsverfahren nach Anspruch 1,
wobei ein Grad des Polynoms 2 oder mehr und 7 oder weniger beträgt.

**3.** Phasenbild-Erfassungsverfahren nach Anspruch 2,
wobei ein Änderungsbetrag in einer Höhe der Bodenfläche des konkaven Abschnitts in dem Phasenbild 10 $\mu$m oder mehr beträgt.

**4.** Phasenbild-Erfassungsverfahren nach einem der Ansprüche 1 bis 3,
wobei das Objekt eine Zelle oder ein Zellaggregat ist.

**5.** Phasenbild-Erfassungsverfahren nach Anspruch 4,
wobei die Zelle oder das Zellaggregat ein befruchtetes Ei eines Tieres ist.

**6.** Phasenbild-Erfassungsverfahren nach Anspruch 4, ferner umfassend:
Erfassen eines Phasenbildes der Zelle oder des Zellaggregats, das in einem Zustand, in dem sie in dem konkaven Abschnitt aufgenommen sind, kultiviert wird.

**7.** Quantitatives Daten-Erfassungsverfahren unter Verwendung des Phasenbild-Erfassungsverfahrens nach Anspruch 4, wobei das quantitative Daten-Erfassungsverfahren umfasst:
Ableiten von quantitativen Daten, die einen Zustand der Zelle oder des Zellaggregats angeben, auf der Grundlage des verarbeiteten Phasenbildes.

**Revendications**

**1.** Procédé d'acquisition d'image de phase comprenant :

acquérir une image d'hologramme d'un objet dans un état où l'objet est logé dans une partie concave prévue dans un conteneur avec une surface inférieure de la partie concave étant courbée ;
générer une image de phase à partir de l'image d'hologramme ; et
effectuer un traitement de soustraction d'une composante de phase dérivée d'un polynôme indiquant une forme de la surface inférieure de la partie concave à partir de l'image de phase pour acquérir une image de phase traitée.

**2.** Procédé d'acquisition d'image de phase selon la revendication 1,
dans lequel un degré du polynôme est de 2 ou plus et de 7 ou moins.

**3.** Procédé d'acquisition d'image de phase selon la revendication 2,
dans lequel une quantité de changement d'une hauteur de la surface inférieure de la partie concave dans l'image de phase est de 10 $\mu$m ou plus.

**4.** Procédé d'acquisition d'image de phase selon l'une quelconque des revendications 1 à 3,
dans lequel l'objet est une cellule ou un agrégat cellulaire.

**5.** Procédé d'acquisition d'image de phase selon la revendication 4,
dans lequel la cellule ou l'agrégat cellulaire est un œuf fécondé d'un animal.

**6.** Procédé d'acquisition d'image de phase selon la revendication 4, comprenant en outre :
acquérir une image de phase de la cellule ou de l'agrégat cellulaire cultivé dans un état d'être logé dans la partie concave.

**7.** Procédé d'acquisition de données quantitatives utilisant le procédé d'acquisition d'image de phase selon la revendication 4, le procédé d'acquisition de données quantitatives comprenant :
dériver des données quantitatives indiquant un état de la cellule ou de l'agrégat cellulaire sur la base de l'image de phase traitée.

# FIG. 1

# FIG. 2

# FIG. 3

# FIG. 4

# FIG. 5

POSITION IN HEIGHT DIRECTION

—— ACTUAL MEASUREMENT
— — — SECOND-DEGREE POLYNOMIAL

POSITION IN PLANE DIRECTION

# FIG. 6A

THIRD-DEGREE
BG CORRECTION

## FIG. 6B

FIFTH-DEGREE
BG CORRECTION

## FIG. 6C

SEVENTH-DEGREE
BG CORRECTION

# FIG. 7

COMPARATIVE EXAMPLE

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2018516591 A

### Non-patent literature cited in the description

- **LAI XIAOMIN et al.** Digital holographic phase imaging with aberrations totally compensated. *BIO-MEDICAL OPTICS EXPRESS*, 01 January 2019, vol. 10 (1), 283